(19) Europäisches Patentamt
European Patent Office
Office européen des brevets

(11) Publication number: **0 307 220 A2**

(12) # EUROPEAN PATENT APPLICATION

(21) Application number: **88308344.6**

(22) Date of filing: **09.09.88**

(51) Int.Cl.[4]: **C 07 D 493/22**
A 01 N 43/90, A 61 K 31/35
//(C07D493/22,313:00,311:00,
311:00,307:00)

(30) Priority: **11.09.87 GB 8721371**

(43) Date of publication of application:
**15.03.89 Bulletin 89/11**

(84) Designated Contracting States:
**AT BE CH DE ES FR GB GR IT LI LU NL SE**

(71) Applicant: **AMERICAN CYANAMID COMPANY**
**One Cyanamid Plaza**
**Wayne New Jersey 07470 (US)**

(72) Inventor: **Ramsay, Michael V. J.**
**157 Kings Road**
**South Harrow Middlesex (GB)**

**Howes, Peter D.**
**31 Buckland Rise**
**Pinner Middlesex (GB)**

**Bell, Richard**
**182 Long Drive**
**South Ruislip Middlesex (GB)**

**Tiley, Edward P.**
**22 South Close**
**Village Way Pinner Middlesex (GB)**

**Sutherland, Derek R.**
**41 Milton Fields**
**Chalfont St Giles Buckinghamshire (GB)**

(74) Representative: **Skailes, Humphrey John et al**
**Frank B. Dehn & Co. Imperial House 15-19 Kingsway**
**London WC2B 6UZ (GB)**

(54) **Macrolide compounds.**

(57) Compounds of formula (I)

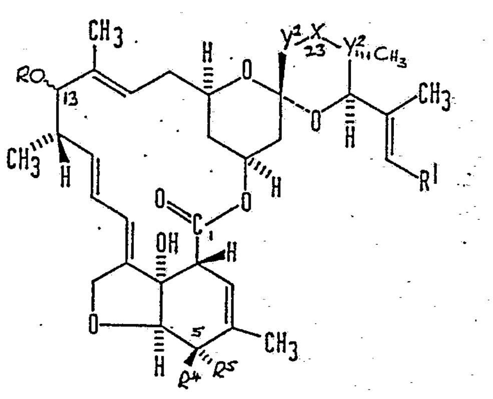

(I)

wherein R represents a hydrogen atom or a silyl group; $R^1$ represents a methyl, ethyl or isopropyl group; $Y^1$ is $-CH_2-$, $Y^2$ is -CH- and X represents $-C(R^2)(R^3)-$[where $R^2$ represents a hydrogen atom or a group $OR^6$ (where $OR^6$ is a hydroxy group or a substituted hydroxyl group having up to 25 carbon atoms) and $R^3$ represents a hydrogen atom, or $R^2$ and $R^3$ together with the carbon atom to which they are attached represents C=O, $C=CH_2$ or $C=NOR^7$ (where $R^7$ represents a hydrogen atom or a $C_{1-8}$ alkyl group and the group $C=NOR^7$ is in the E configuration)] or $-Y^1-X-Y^2-$ represents $-CH=CH-CH-$ or $-CH_2-C\equiv C-$;
$R^4$ represents a group $OR^6$ as defined above and $R^5$ represents a hydrogen atom or $R^4$ and $R^5$ together with the carbon atom to which they are attached represents C=O.
These compounds may be used for controlling insect, acarine, nematode or other pests.

EP 0 307 220 A2

## Description

### Macrolide Compounds

This invention relates to novel macrolide compounds and to processes for their preparation.

Thus, in one aspect, the invention particularly provides the compounds of formula (I):

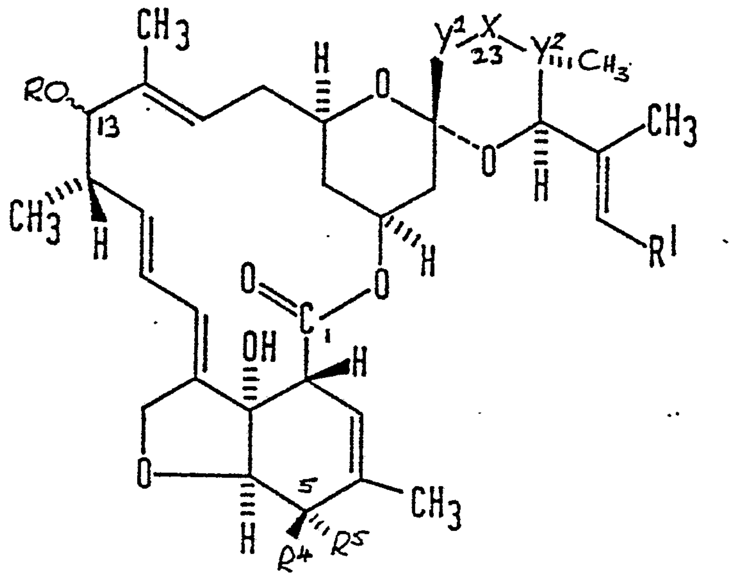

(I)

wherein R represents a hydrogen atom or a silyl group;

$R^1$ represents a methyl, ethyl or isopropyl group;

$Y^1$ is $-CH_2-$, $Y^2$ is -CH- and X represents $-C(R^2)(R^3)-$ [where $R^2$ represents a hydrogen atom or a group $OR^6$ (where $OR^6$ is a hydroxy group or a substituted hydroxyl group having up to 25 carbon atoms) and $R^3$ represents a hydrogen atom, or $R^2$ and $R^3$ together with the carbon atom to which they are attached represents $C=O$, $C=CH_2$ or $C=NOR^7$ (where $R^7$ represents a hydrogen atom or a $C_{1-8}$ alkyl group and the group $C=NOR^7$ is in the E configuration)] or $-Y^1-X-Y^2-$ represents $-CH=CH-CH-$ or $-CH_2-C=C-$; $R^4$ represents a group $OR^6$ as defined above and $R^5$ represents a hydrogen atom or $R^4$ and $R^5$ together with the carbon atom to which they are attached represents $C=O$.

The 13-position in the compounds of formula (I) is a chiral centre and the line joining the hydroxyl group to the carbon atom at the 13-position denotes that the present invention encompasses both (R) and (S) compounds of formula (I) as well as mixtures thereof.

Compounds of formula (I) are of use as antibiotics. The compounds of the invention are also useful as intermediates in the preparation of further active compounds. When the compounds of formula (I) are to be used as intermediates, the group $-OR^6$ when present will often be a protected hydroxy group and/or the group R will be a silyl group and the invention particularly includes such compounds.

When the group R in compounds of formula (I) is a silyl group it may for example carry three groups which may be the same or different, selected from alkyl, alkenyl, alkoxy, cycloalkyl, aralkyl, aryl and aryloxy groups. Such groups may be as defined below for the group $R^8$ and particularly include t-butyl and phenyl groups. Particular examples of such silyl groups are t-butyldimethylsilyl and diphenylmethylsilyl.

The group $R^6$ when present in compounds of formula (I) may represent an acyl group e.g. a group of the formula $R^8CO-$ or $R^8OCO-$(where $R^8$ is an aliphatic, araliphatic or aromatic group, for example an alkyl, cycloalkyl, aralkyl or aryl group), a group $-COCO_2H$, a group $R^9$ which is as defined above for $R^8$, a group $R^{10}SO_2-$ (where $R^{10}$ is a $C_{1-4}$ alkyl or $C_{6-10}$ aryl group), or a silyl group.

Where $R^8$ or $R^9$ are alkyl groups, they may be for example $C_{1-8}$ alkyl groups, e.g. methyl, ethyl or propyl, which alkyl groups may also be substituted. Where $R^8$ is a substituted alkyl group it may be substituted by, for example, one or more halogen atoms (e.g. chlorine or bromine atoms), or a carboxy, $C_{1-4}$ alkoxy (e.g. methoxy, ethoxy), phenoxy or silyloxy group. Where $R^9$ is a substituted alkyl group it may be substituted by one or more halogen atoms (e.g. chlorine or

Where $R^8$ or $R^9$ are aralkyl groups, they preferably have 1-6 carbon atoms in the alkyl moiety, and the aryl group(s) may be carbocyclic and preferably contain 4-15 carbon atoms e.g. phenyl.

Where $R^8$ and $R^9$ are aryl groups, they may be carbocyclic and preferably have 4-15 carbon atoms e.g. phenyl.

When $R^6$ is a group $R^{10}SO_2-$, it may be for example a methylsulphonyl or p-toluenesulphonyl group.

When $R^6$ represents a silyl group or $R^8$ contains a silyloxy substituent, the silyl moiety may be as defined

above for the group R.

Where $R^7$ represents a $C_{1-8}$ alkyl group, it may be for example a methyl, ethyl, n-propyl, i-propyl, n-butyl, i-butyl or t-butyl group, and is preferably a methyl group.

In the compounds of formula (I) $R^1$ preferably represents an isopropyl group.

$R^4$ preferably represents a hydroxyl group or an acyloxy, especially an acetoxy group.

An important group of compounds of formula (I) is that in which $Y^1$ is $-CH_2-$, $Y^2$ is -CH-, and X represents $-C(R^2)(R^3)-$. Particularly important compounds of this type are those in which $R^4$ is a group $OR^6$ and $R^5$ is a hydrogen atom.

In a particular preference, R in the compounds of formula (I) is a silyl group, or especially, a hydrogen atom, $R^1$ is an isopropyl group, $Y^1$ is $-CH_2-$, $Y^2$ is -CH-, X is $-C(R^2)(R^3)$, in which $R^2$ is a hydroxy, ethoxy or acetyloxy group and $R^3$ is hydrogen atom, or $R^2$ and $R^3$ together with the carbon atom to which they are attached represents $C=O$, $C=CH_2$ or $C=NOCH_3$ (where the group $C=NOCH_3$ is in the E configuration), or $R^2$ and $R^3$ each represents a hydrogen atom; and $R^4$ is a hydroxy, methoxy or acetoxy group and $R^5$ is a hydrogen atom.

Important active compounds according to the invention are those of formula (I) in which:
R is a hydrogen atom, $R^1$ is an isopropyl group, $Y^1$ is $-CH_2-$, $Y^2$ is -CH-, X represents $-CH_2-$, $R^4$ is a hydroxyl group and $R^5$ is a hydrogen atom; and
R is a hydrogen atom, $R^1$ is an isopropyl group, $Y^1$ is $-CH_2-$, $Y^2$ is -CH-, X represents $-CH_2-$, $R^4$ is an acetoxy group and $R^5$ is a hydrogen atom.

As indicated previously, compounds according to the invention may be of use as antibiotics and/or as intermediates for the preparation of further active compounds. When the compounds of the invention are to be used as intermediates, the $R^4$ group may serve as a protecting group. It will be appreciated that such a protecting group should have the minimum of additional functionality to avoid further sites of reaction and should be selectively removable. Examples of groups serving as hydroxyl protecting groups are well known and are described, for example, in "Protective Groups in Organic Synthesis" by Theodora W. Greene. (Wiley-Interscience, New York 1981) and "Protective Groups in Organic Chemistry" by J F W McOmie (Plenum Press, London, 1973). Examples of suitable $R^4$ protecting groups include phenoxyacetyl, silyloxyacetyl, (e.g. trimethylsilyloxyacetyl and t-butyldimethylsilyloxyacetyl), and silyl such as trimethylsilyl and t-butyldimethylsilyl. Compounds of the invention containing such groups will primarily be of use as intermediates. Other groups, such as acetyl, may serve as protecting groups, but may also be present in final active compounds.

Compounds of the invention have antibiotic activity e.g. antihelminthic activity, for example against nematodes, and in particular, anti-endoparasitic and anti-ectoparasitic activity.

Ectoparasites and endoparasites infect humans and a variety of animals and are particularly prevalent in farm animals such as pigs, sheep, cattle, goats and poultry, horses and domestic animals such as dogs and cats. Parasitic infection of livestock, leading to anaemia, malnutrition and weight loss is a major cause of economic loss throughout the world.

Examples of genera of endoparasites infecting such animals and/or humans are Ancylostoma, Ascaridia, Ascaris, Aspicularis, Brugia, Bunostomum, Capillaria, Chabertia, Cooperia, Dictyocaulus, Dirofilaria, Dracunculus, Enterobius, Haemonchus, Heterakis, Loa, Necator, Nematodirus, Nematospiroides (Heligomoroides), Nippostrongylus, Oesophagostomum, Onchocerca, Ostertagia, Oxyuris, Parascaris, Strongylus, Strongyloides, Syphacia, Toxascaris, Toxocara, Trichonema, Trichostrongylus, Trichinella, Trichuris, Uncinaria and Wuchereria.

Examples of ectoparasites infecting animals and/or humans are arthropod ectoparasites such as biting insects, blowfly, fleas, lice, mites, sucking insects, ticks and other dipterous pests.

Examples of genera of such ectoparasites infecting animals and/or humans are Ambylomma, Boophilus, Chorioptes, Culliphore, Demodex, Damalinia, Dermatobia, Gastrophilus, Haematobia, Haematopinus, Haemophysalis, Hyaloma, Hypoderma, Ixodes, Linognathus, Lucilia, Melophagus, Oestrus, Otodectes, Psorergates, Psoroptes, Rhipicephalus, Sarcoptes, Stomoxys and Tabanus.

The compounds of the invention are therefore of use in treating animals and humans with endoparasitic and/or ectoparasitic infections.

The antibiotic activity of compounds of the invention may, for example, be demonstrated by their activity in vitro against free living nematodes e.g. Caenorhabiditis elegans.

Furthermore, compounds of the invention are of use as anti-fungals, for example, against strains of Candida sp. such as Candida albicans and Candida glabrata and against yeast such as Saccharomyces carlsbergensis.

Compounds of the invention are also of use in combating insect, acarine and nematode pests in agriculture, horticulture, forestry, public health and stored products. Pests of soil and plant crops, including cereals (e.g. wheat, barley, maize and rice) vegetables (e.g. soya), fruit (e.g. apples, vines and citrus) as well as root crops (e.g. sugarbeet, potatoes) may usefully be treated. Particular examples of such pests are fruit mites and aphids such as Aphis fabae, Aulacorthum circumflexum, Myzus persicae, Nephotettix cincticeps, Nilparvata lugens, Panonychus ulmi, Phorodon humuli, Phyllocoptruta oleivora, Tetranychus urticae and members of the genera Trialeuroides; nematodes such as members of the genera Aphelencoides, Globodera, Heterodera, Meloidogyne and Panagrellus; lepidoptera such as Heliothis, Plutella and Spodoptera; grain weevils such as Anthonomus grandis and Sitophilus granarius; flour beetles such as Tribolium castaneum; flies such as Musca domestica; fire ants; leaf miners; Pear psylla; Thrips tabaci; cockroaches such as Blatella germanica and Periplaneta americana and mosquitoes such as Aedes aegypti.

According to the invention we therefore provide compounds of formula (I) as defined above, which may be used as antibiotics. In particular, they may be used in the treatment of animals and humans with endoparasitic, ectoparasitic and/or fungal infections and in agriculture, horticulture, or forestry as pesticides to combat insect, acarine and nematode pests. They may also be used generally as pesticides to combat or control pests in other circumstances, e.g. in stores, buildings or other public places or location of the pests. In general the compounds may be applied either to the host (animal or human or plants or vegetation) or to the pests themselves or a locus thereof.

Compounds of the invention may be formulated for administration in any convenient way for use in veterinary or human medicine and the invention therefore includes within its scope pharmaceutical compositions comprising a compound in accordance with the invention adapted for use in veterinary or human medicine. Such compositions may be presented for use in conventional manner with the aid of one or more suitable carriers or excipients. The compositions of the invention include those in a form especially formulated for parenteral (including intramammary administration), oral, rectal, topical, implant, ophthalmic, nasal or genito-urinary use.

The compounds according to the invention may be formulated for use in veterinary or human medicine by injection and may be presented in unit dose form, in ampoules, or other unit-dose containers, or in multi-dose containers, if necessary with an added preservative. The compositions for injection may be in the form of suspensions, solutions, or emulsions in oily or aqueous vehicles, and may contain formulatory agents such as suspending, stabilising, solubilising and/or dispersing agents. Alternatively the active ingredient may be in sterile powder form for reconstitution with a suitable vehicle, e.g. sterile, pyrogen-free water, before use. Oily vehicles include polyhydric alcohols and their esters such as glycerol esters, fatty acids, vegetable oils such as arachis oil or cottonseed oil, mineral oils such as liquid paraffin, and ethyl oleate and other similar compounds. Other vehicles such as propylene glycol may also be used.

Compositions for veterinary medicine may also be formulated as intramammary preparations in either long acting or quick-release bases and may be sterile solutions or suspensions in aqueous or oily vehicles optionally containing a thickening or suspending agent such as soft or hard paraffins, beeswax, 12-hydroxy stearin, hydrogenated castor oil, aluminium stearates, or glyceryl monostearate. Conventional non-ionic, cationic or anionic surface active agents may be used alone or in combination in the composition.

The compounds of the invention may also be presented for veterinary or human use in a form suitable for oral administration, for example in the form of solutions, syrups or suspensions, or a dry powder for constitution with water or other suitable vehicle before use, optionally with flavouring and colouring agents. Solid compositions such as tablets, capsules, lozenges, pills, boluses, powder, pastes, granules, bullets or premix preparations may also be used. Solid and liquid compositions for oral use may be prepared according to methods well known in the art. Such compositions may also contain one or more pharmaceutically acceptable carriers and excipients which may be in solid or liquid form. Examples of suitable pharmaceutically acceptable carriers for use in solid dosage forms include binding agents (e.g. pregelatinised maize starch, polyvinylpyrrolidone or hydroxypropyl methylcellulose); fillers (e.g. lactose, micro-crystalline cellulose or calcium phosphate); lubricants (e.g. magnesium stearate, talc or silica); disintegrants (e.g. potato starch or sodium starch glycollate); or wetting agents (e.g. sodium lauryl sulphate). Tablets may be coated by methods well known in the art.

Examples of suitable pharmaceutically acceptable additives for use in liquid dosage forms include suspending agents (e.g. sorbitol syrup, methyl cellulose or hydrogenated edible fats); emulsifying agents (e.g. lecithin or acacia); non-aqueous vehicles (e.g. almond oil, oily esters or ethyl alcohol); and preservatives (e.g. methyl or propyl p-hydroxybenzoates or sorbic acid); stabilising and solubilising agents may also be includeed.

Pastes for oral administration may be formulated according to methods well known in the art. Examples of suitable pharmaceutically acceptable additives for use in paste formulations include suspending or gelling agents e.g. aluminium distearate or hydrogenated castor oil; dispersing agents e.g. polysorbates, non-aqueous vehicles e.g. arachis oil or oily esters; stabilising and solubilising agents. The compounds of the invention may also be administered in veterinary medicine by incorporation thereof into animals daily solid or liquid dietary intake, e.g. as part of the daily animal feed or drinking water.

The compounds of the invention may also be administered orally in veterinary medicine in the form of a liquid drench such as a solution, suspension or dispersion of the active ingredient together with a pharmaceutically acceptable carrier or excipient.

The compounds of the invention may also, for example, be formulated as suppositories e.g. containing conventional suppository bases for use in veterinary or human medicine or as pessaries e.g. containing conventional pessary bases.

Compounds according to the invention may be formulated for topical administration, for use in veterinary and human medicine, as ointments, creams, lotions, shampoos, powders, pessaries, sprays, dips, aerosols, drops (e.g. eye or nose drops) or pour-ons. Ointments and creams may, for example, be formulated with an aqueous or oily base with the addition of suitable thickening and/or gelling agents. Ointments for administration to the eye may be manufactured in a sterile manner using sterilised components. Pour-ons may, for example, be formulated for veterinary use in oils containing organic solvents, optionally with formulatory agents e.g. stabilising and solubilising agents.

Lotions may be formulated with an aqueous or oily base and will in general also contain one or more

emulsifying agents, stabilising agents, dispersing agents, suspending agents, thickening agents, or colouring agents.

Powders may be formed with the aid of any suitable powder base. Drops may be formulated with an aqueous or non aqueous base also comprising one or more dispersing agents, stabilising agents, solubilising agents or suspending agents. They may also contain a preservative.

For topical administration by inhalation the compounds according to the invention may be delivered for use in veterinary or human medicine in the form of an aerosol spray presentation or an insufflator.

The compounds of the invention may be administered in combination with other pharmaceutically active ingredients.

The total daily dosages of compounds of the invention employed in both veterinary and human medicine will suitably be in the range 1-2000μg/kg bodyweight, preferably from 50-1000μg/kg and these may be given in divided doses, e.g. 1-4 times per day.

The compounds according to the invention may be formulated in any convenient way for horticultural or agricultural use and the invention therefore includes within its scope compositions comprising a compound according to the invention adapted for horticultural or agricultural use. Such formulations include dry or liquid types, for example dusts, including dust bases or concentrates, powders, including soluble or wettable powders, granulates, including microgranules and dispersible granules, pellets, flowables, emulsions such as dilute emulsions or emulsifiable concentrates, dips such as root dips and seed dips, seed dressings, seed pellets, oil concentrates, oil solutions, injections e.g. stem injections, sprays, smokes and mists.

Generally such formulations will include the compound in association with a suitable carrier or diluent. Such carriers may be liquid or solid and designed to aid the application of the compound either by way of dispersing it where it is to be applied or to provide a formulation which can be made by the user into a dispersible preparation. Such formulations are well known in the art and may be prepared by conventional methods such as, for example by blending and/or grinding of the active ingredients(s) together with the carrier or diluent, e.g. solid carrier, solvent or surface active agent.

Suitable solid carriers, for use in formulations such as dusts, granulates and powders may be selected from for example natural mineral fillers, such as diatomite, talc, kaolinite, montmorillonite pyrophyllite or attapulgite. Highly dispersed silicic acid or highly dispersed absorbent polymers may, if desired, be included in the composition. Granulated adsorptive carriers which may be used may be porous (such as pumice, ground brick, sepiolite or bentonite) or non-porous (such as calcite or sand). Suitable pregranulated materials which may be used and which may be organic or inorganic include dolomite and ground plant residues.

Suitable solvents for use as carriers or diluents include aromatic hydrocarbons, aliphatic hydrocarbons, alcohols and glycols or ethers thereof, ester, ketones, acid amides, strongly polar solvents, optionally epoxidized vegetable oils and water.

Conventional non-ionic, cationic or anionic surface-active agents, e.g. ethoxylated alkyl phenols and alcohols, alkali metal or alkaline earth metal salts of alkyl benzene sulphonic acids, lignosulphonic acids or sulphosuccinic acids or sulphonates of polymeric phenols which have good emulsifying, dispersing and/or wetting properties may also be used either alone or in combination in the compositions.

Stabilizers, anti-caking agents, anti-foaming agents, viscosity regulators, binders and adhesives, photostabilisers as well as fertilizers, feeding stimulants or other active substances may, if desired, be included in the compositions. The compounds of the invention may also be formulated in admixture with other insecticides, acaricides and nematicides.

In the formulations, the concentration of active material is generally from 0.01 to 99% and more preferably between 0.01% and 40% by weight.

Commercial products are generally provided as concentrated compositions to be diluted to an appropriate concentration, for example from 0.001 to 0.0001% by weight, for use

The compounds according to the invention may be prepared by a number of processes as described in the following where R, $R^1$, $R^2$, $R^3$, $R^4$, $R^5$, $R^6$, $R^7$, X, $Y^1$ and $Y^2$ are as defined for general formula (I) unless specified otherwise. In some of these processes it may be necessary to protect a hydroxyl group at the 5-, 13- and/or 23-position in the starting material prior to effecting the reaction described. In such cases it may then be necessary to deprotect the same hydroxyl group once the reaction has occurred to obtain the desired compound of the invention. Conventional methods of protection and deprotection may be used, for example as described in the aforementioned books by Greene and McOmie.

According to one process (A), a compound of formula (I) in which the 13-hydroxyl group is in the (R)-configuration and $R^4$ is as defined for formula (I) but is not a hydroxyl or silyloxy group may be prepared by oxidising a compound of formula (II)

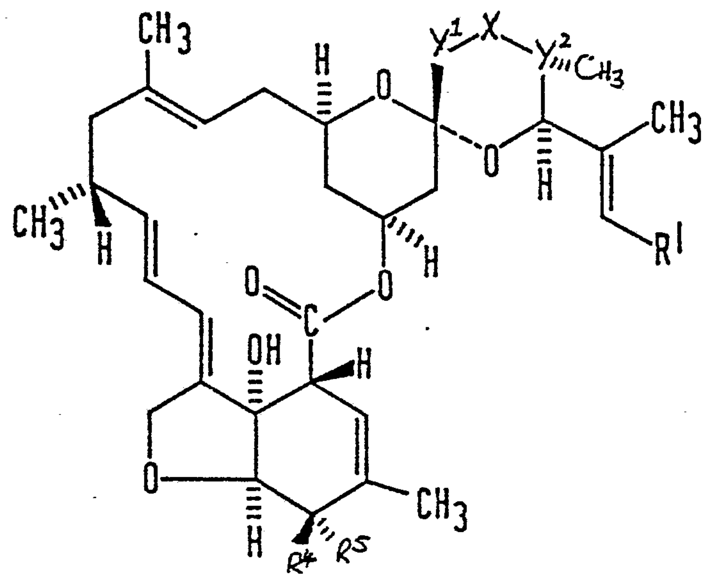

(II)

wherein $R^4$ is as just defined.

The oxidation may be effected for example with an oxidising agent such as selenium dioxide, preferably in the presence of an activator such as a peroxide, e.g. tert-butyl hydroperoxide. The reaction may conveniently be effected in an inert solvent such as a halogenated hydrocarbon e.g. dichloromethane, an ester, e.g. ethyl acetate or an ether, e.g. tetrahydrofuran, at a temperature in the range of 0° to 50°C, preferably at room temperature.

In another process (B), a compound of formula (I) in which the 13-hydroxyl group is in the (S)-configuration may be prepared by reducing a compound of formula (III)

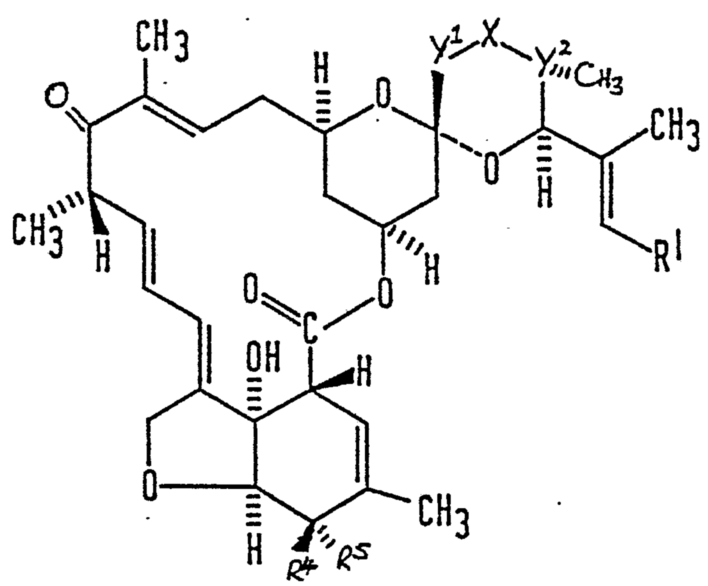

(III)

The reduction may be effected for example using a reducing agent such as a borohydride, for example an alkali metal borohydride such as sodium borohydride or a lithium alkoxyaluminium hydride such as lithium tributoxyaluminium hydride.

The reaction involving a borohydride reducing agent takes place in the presence of a solvent such as an alkanol e.g. isopropyl alcohol or isobutyl alcohol conveniently at a temperature in the range of -30° to +80°C e.g. at 0°C. The reaction involving a lithium alkoxyaluminium hydride takes place in the presence of a solvent such as an ether e.g. tetrahydrofuran or dioxan conveniently at a temprature in the range of -78° to 0°C e.g. at -78°C.

Intermediate compounds of formula (III) in which $R^4$ is a substituted hydroxyl group may be prepared from

compounds of formula (I) wherein the 13-hydroxyl group is in the (R)-configuration by oxidation. Suitable oxidising agents for the conversion include dialkylsulphoxides e.g. dimethylsulphoxide, in the presence of an activating agent such as N,N′-dicyclohexylcarbodiimide or an acyl halide, e.g. oxalyl chloride. The reaction may conveniently be effected in a suitable solvent such as a halogenated hydrocarbon e.g. methylene chloride at a temperature in the range of -80° to +50°C.

Intermediates of formula (III) in which $R^4$ is a hydroxyl group may be prepared from the corresponding compounds of formula (III) in which $R^4$ is a substituted hydroxyl group using the methods discussed below for the preparation of compounds of formula (I) in which $R^4$ is a hydroxyl group.

The intermediates of formula (III) are novel compounds and form a further aspect of the invention.

In yet another process (C), a compound of formula (I) in which $R^4$ is a hydroxyl group may be prepared from a corresponding compound of formula (I) in which $R^4$ is a substituted hydroxyl group by conversion of the group $R^6$ to a hydrogen atom. The conversion will usually be carried out in the context of removing a protecting group such as referred to above.

Thus, deprotection of the compounds of the invention in which $R^4$ represents a protected hydroxyl group can be effected by conventional methods, for example those extensively described in the aforementioned textbooks of McOmie and Greene. Thus, for example, when $R^4$ is an acyloxy group such as an acetoxy group the acetyl group may be removed by basic hydrolysis, e.g. using sodium or potassium hydroxide or ammonia in an aqueous alcohol such as methanol to yield the compound of formula (I) in which $R^4$ is a hydroxyl group.

In a further process (D), the compounds of the invention in which $R^4$ is a substituted hydroxyl group may generally be prepared by reacting the corresponding 5-hydroxy compound with reagents serving to form a substituted hydroxyl group.

The reaction will in general be an acylation, sulphonylation, etherification, or silylation.

Thus, for example, acylation may be effected using an acylating agent such as an acid of formula $R^8COOH$ or a reactive derivative thereof, such as an acid halide (e.g. acid chloride), anhydride or activated ester, or a reactive derivative of a carbonic acid $R^8OCOOH$.

Acylations employing acid halides and anhydrides may if desired be effected in the presence of an acid binding agent such as a tertiary amine (e.g. triethylamine, dimethylaniline or pyridine), inorganic bases (e.g. calcium carbonate or sodium bicarbonate), and oxiranes such as lower 1,2-alkylene oxides (e.g. ethylene oxide or propylene oxide) which bind hydrogen halide liberated in the acylation reaction.

Acylations employing acids are desirably conducted in the presence of a condensing agent, for example a carbodiimide such as N,N′-dicyclohexylcarbodiimide or N-ethyl-N′γ-dimethylaminopropylcarbodiimide; a carbonyl compound such as carbonyldiimidazole; or an isoxazolium salt such as N-ethyl-5-phenylisoxazolium perchlorate.

An activated ester may conveniently be formed <u>in situ</u> using, for example, 1-hydroxybenzotriazole in the presence of a condensing agent as set out above. Alternatively, the activated ester may be preformed.

The acylation reaction may be effected in aqueous or non-aqueous reaction media, conveniently at a temperature in the range -20° to +100°C, e.g. -10° to +50°C.

Compounds in which $R^4$ is a group $-OCOCO_2H$ may be prepared by reation with oxalyl chloride, optionally in the presence of an acid scavenger.

Sulphonylation may be effected with a reactive derivative of a sulphonic acid $R^{10}SO_3H$ such as a sulphonyl halide, for example a chloride $R^{10}SO_2Cl$ or a sulphonic anhydride. The sulphonylation is preferably effected in the presence of a suitable acid binding agent as described above.

Etherification may be effected using a reagent of formula $R^9Y$ (where $R^9$ is as previously defined and Y represents a leaving group such as chlorine, bromine or iodine atom or a hydrocarbylsulphonyloxy group, such as mesyloxy or tosyloxy, or a haloalkanoyloxy group such as dichloroacetoxy). The reaction may be carried out by formation of a magnesium alkoxide using a Grignard reagent such as a methylmagnesium halide e.g. methylmagnesium iodide or using a trialkylsilylmethylmagnesium halide e.g. trimethylsilylmethylmagnesium chloride followed by treatment with the reagent $R^9Y$.

Alternatively, the reaction may be effected in the presence of a silver salt such as silver oxide, silver perchlorate, silver carbonate or silver salicylate or mixtures thereof, and this system may be particularly appropriate when etherification is carried out using an alkyl halide (e.g. methyl iodide).

Etherification may conveniently be effected in a solvent such as an ether e.g. diethyl ether.

Solvents which may be employed in the above reactions include ketones (e.g. acetone), amides (e.g. N,N-dimethylformamide, N,N-dimethylacetamide or hexamethylphosporamide), ethers (e.g. cyclic ethers such as tetrahydrofuran or dioxan, and acyclic ethers such as dimethoxyethane or diethylether), nitriles (e.g. acetonitrile), hydrocarbons such as halogenated hydrocarbons (e.g. methylene chloride), and esters such as ethyl acetate, as well as mixtures of two or more such solvents.

Silylation may be effected by reaction with a silyl halide (e.g. chloride), advantageously in the presence of a base such as imidazole triethylamine or pyridine, using a solvent such as dimethylformamide.

In a further process (E) according to the invention, a compound of formula (I) in which X is $-C(R^2)R^3)-$ and $R^2$ and $R^3$ together with the carbon atom to which they are attached represents $C=O$ and/or $R^4$ and $R^5$ together with the carbon atom to which they are attached represents $C=O$, may be prepared by oxidising a corresponding compound of formula (I) wherein $R^2$ is a hydroxyl group and $R^3$ is a hydrogen atom and/or $R^4$ is a silyloxy group and $R^5$ is a hydrogen atom. The reaction may be effected with an oxidising agent serving to convert a secondary hydroxyl group to an oxo group, whereby a compound of formula (I) is produced.

Suitable oxidising agents include quinones in the presence of water, e.g. 2,3-dichloro-5,6-dicyano-1,4-benzoquinone or 2,3,5,6-tetrachloro-1,4-benzoquinone; a chromium (VI) oxidising agent, e.g. pyridinium dichromate or chromium trioxide in pyridine; a manganese (IV) oxidising agent, e.g. manganese dioxide in dichloromethane; an N-halosuccinimide, e.g. N-chlorosuccinimide or N-bromosuccinimide; a dialkylsulphoxide e.g. dimethylsulphoxide, in the presence of an activating agent such as N,N′-dicyclohexylcarbodiimide or an acyl halide, e.g. oxalyl choride; or a pyridine-sulphur trioxide complex.

The reaction may conveniently be effected in a suitable solvent which may be selected from a ketone, e.g. acetone; an ether, e.g. diethyl ether, dioxan or tetrahydrofuran; a hydrocarbon, e.g. hexane; a halogenated hydrocarbon e.g. chloroform or methylene chloride; or an ester, e.g. ethyl acetate or a substituted amide e.g. dimethylformamide. Combinations of such solvents either alone or with water may also be used. The choice of solvent will depend on the oxidising agent used to effect the conversion.

The reaction may be carried out at a temperature of from -80°C to +50°C.

In another process (F) according to the invention a compound of formula (I) in which X is $-C(R^2)(R^3)-$ and $R^2$ and $R^3$ together with the carbon atom to which they are attached represent $C=NOR^7$ may be prepared from the corresponding 23-keto compound of formula (I) in which $R^2$ and $R^3$ together with the carbon atom to which they are attached represent $C=O$ by reaction with a reagent $H_2NOR^7$ (where $R^7$ is as defined previously). The reaction is preferably carried out using about one equivalent of the reagent $H_2NOR^7$.

The reaction may be conveniently carried out at a temperature in the range -20 to +100°C, e.g. -10 to +50°C in a suitable solvent. It is convenient to use the reagent $H_2NOR^7$ in the form of a salt, for example an acid addition salt such as the hydrochloride. When such a salt is employed the reaction may be carried out in the presence of an acid binding agent.

Solvents which may be employed include alcohols (e.g. methanol or ethanol), amides (e.g. N,N-dimethylformamide, N,N-dimethylacetamide or hexamethylphosphoramide), ethers (e.g. cyclic ethers such as tetrahydrofuran or dioxan, and acylic ethers such as dimethoxyethane or diethylether), nitriles (e.g. acetonitrile), sulphones (e.g. sulpholane), and hydrocarbons such as halogenated hydrocarbons (e.g. methylene chloride), as well as mixtures of two or more such solvents. Water may also be employed as a co-solvent.

When two conditions are employed the reaction may conveniently be buffered with an appropriate acid, base or buffer.

Suitable acids include mineral acids, such as hydrochloric or sulphuric acid, and carboxylic acid such as acetic acid. Suitable bases include alkali metal carbonates and bicarbonates such as sodium bicarbonate, hydroxides such as sodium hydroxide, and alkali metal carboxylates such as sodium acetate. A suitable buffer is sodium acetate/acetic acid.

In a further process (G), a compound of formula (I) in which X is a group $C=CH_2$ may be prepared by reaction of a corresponding compound of formula (I) in which X is $C=O$ with an appropriate Wittig reagent e.g. a phosphorane of formula $(R^{11})_3P=CH_2$ (where $R^{11}$ is $C_{1-6}$ alkyl or aryl, e.g. monocyclic aryl such as phenyl). Suitable reaction solvents include ethers such as tetrahydrofuran or diethyl ether or a dipolar aprotic solvent such as dimethylsulphoxide. The reaction may be carried out at any suitable temperature e.g. at 0°C.

Intermediates of formula (II) in which $Y^1$ is $-CH_2-$, X is $-CH_2-$ and $Y^2$ is $-CH-$ may be prepared by reduction of a compound of formula (IV)

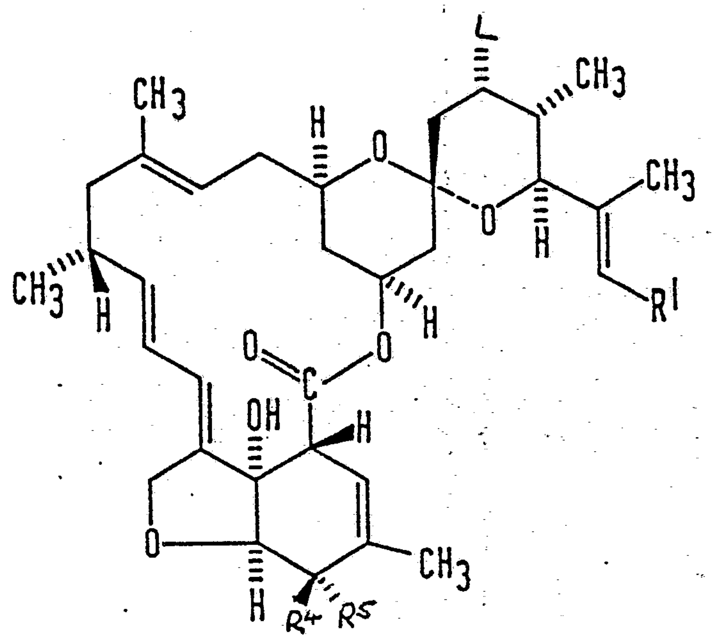

(IV)

[wherein $R^4$ is a substituted hydroxyl group and L is an atom or group removable by reduction, (for example, by homolytic reduction) such as the group $R^{12}OCSO$-, [where $R^{12}$ is $C_{1-6}$ alkyl, aryl such as phenyl, or ($C_{1-6}$alkyl)aryl] such as a p-tolyloxythiocarbonyloxy group.

The reduction may be effected using a reducing agent such as an alkyl tin hydride (e.g. tri-n-butyl tin hydride) in the presence of a radical initiator such as a peroxide, a azobisisobutyronitrile or light.

The reaction may conveniently be effected in a suitable solvent which may be selected from a ketone, e.g. acetone; an ether, e.g. dioxan, a hydrocarbon, e.g. hexane or toluene; a halogenated hydrocarbon e.g. trichlorobenzene; or an ester e.g. ethyl acetate. Combinations of such solvents either alone or with water may also be used.

The reaction may be carried out at a temperature of from 0°-200°C preferably from 20° to 130°C.

Intermediates of formula (IV) may be prepared by reaction of a compound of formula (II) in which $Y^1$ is -$CH_2$-, X is -CH(OH)- and Y is -CH- with a reagent capable of replacing the hydroxyl group in X 23-position with an atom or group L (where L is as just defined).

Suitable reagents which may serve to introduce the moiety L include, for example aryl halothionoformates such as p-tolyl chlorothionoformate. The reaction may be carried out in the presence of a base, for example an amine such as pyridine in a solvent such as a halogenated hydrocarbon e.g. dichloromethane.

The starting materials for this reaction may be prepared by substitution of the 5-hydroxyl group of the corresponding compound in using the acylation, etherification or sulphonylation reactions described above for the preparation of compounds of formula (I).

This last mentioned intermedite may be obtained using the fermentation and isolation methods described in UK Patent Specification 2166436. Other intermediates of formula (II) may be prepared from this compound using the interconversion processes described above for the preparation of compounds of formula (I).

The invention is further illustrated by the following Preparations and Examples wherein the compound of formula (IV) above in which $R^1$ is isopropyl, and $R^4$ and L are OH is referred to as Factor A. Compounds according to the invention are named with respect to Factor A. All temperatures are in °C.

Intermediate 1

5-Acetoxy and 5,23-Diacetoxy Factor A

Factor A (3.0 g) in pyridine (20 ml) at -5° was treated with acetic anhydride (8 ml) and the resulting solution left at 3° for 20h. Benzene (100 ml) was added and the solution concentrated in vacuo. The residual oil was chromatographed over silica using dichloromethane:acetone (40:1) as eluent to give the 5-acetate of Factor A (2.06 g), containing the 5,23-diacetate (10%). The compounds were separated by reverse-phase preparative hplc to give 5-acetoxy Factor A (79% recovery), $\lambda_{max}$ (ethanol) 244.5 nm ($E^1_1$ 462), δ ($CDCl_3$) includes 2.14 (s; 3H), m/z includes 654, 594 and 576, and 5,23-diacetoxy Factor A (6.5% recovery), δ ($CDCl_3$) include 2.01 (s; 3H) and 2.13 (s; 3H), m/z include 696 and 636.

Intermediate 2

Factor A 5-acetate 23-p-tolylthionocarbonate

A solution of Factor A 5-acetate (4g) in dry dichloromethane (50 ml) and dry pyridine (4.9ml) under nitrogen was treated with p-tolylchlorothionoformate (3.7ml) dropwise over a period of 10 minutes. The resulting dark solution was stirred at room temperature for 45h. The solution was diluted with dichloromethane (200ml), washed successively with 2N-hydrochloric acid, saturated sodium bicarbonate solution, water and saturated brine (2x200ml), then dried ($MgSO_4$) and solvent was evaporated to give a dark green foam. This was redissolved in ethyl acetate (200ml) and treated with activated charcoal. Filtration followed by evaporation afforded a pale green foam which was chromatographed on silica [Merck Kieselgel 60, particle size 0.040-0.063mm, mesh 230-400 under atmospheric pressure eluting with hexane-ethyl acetate (2:1)], to give the title compound as a pale yellow foam (3.946g). $\nu_{max}$ ($CHBr_3$) 3620-3340 (OH) 1731 (acetate), 1710 $cm^{-1}$ (carbonyl), δ ($CDCl_3$) includes 6.81 (d,6Hz,3H), 2.16 (s,3H), 2.36 (s,3H), 3.34 (m,1H), 6.99 (d,9Hz,2H), 7.20 (d,9Hz,2H).

Intermediate 3

23-deoxy-Factor A 5-acetate

A solution of Factor A 5-acetate 23-p-tolylthionocarbonate (10.194g) in dry toluene (100ml) was heated to reflux under nitrogen and treated with α-azo-bis-isobutyronitrile (509mg). A solution of tri-n-butyltin hydride (10.25ml) in dry toluene (60ml) was added dropwise over a period of 25 minutes, maintaining the reflux conditions. The mixture was stirred for a further 25mins, then cooled to room temperature and solvent was evaporated to give a yellow oil. This was dissolved in acetonitrile (600ml) and washed with hexane. The solvent was evaporated to give a white foam which was chromatographed on silica (Merck Kieselgel 60, particle size 0.040-0.063mm, mesh 230-400) eluting with hexane-ethyl acetate (4:1), to afford the title compound (2.442g), $[\alpha]_D^{22}$ + 144° (c 0.43 chloroform), $\nu_{max}$ ($CHBr_3$) 3420-3340 (OH), 1732 (acetate), 1710$cm^{-1}$ (carbonyl), δ ($CDCl_3$) includes 0.68 (d,5Hz,3H), 2.16 (s,3H), 3.32 (m, 1H).

Intermediate 4

13-Oxo-23-desoxy Factor A, 5-acetate

A solution of dimethyl sulphoxide (92 µl) in dichloromethane (1 ml) was added dropwise over 2 min to a solution of oxalyl chloride (57 µl) in dichloromethane (2ml) at -50°, under an atmosphere of nitrogen. After 5 min a solution of the compound of Example 1 (213 mg) in dichloromethane (3 ml) was added dropwise over 2 min at -50°. After 30 min at -50° to -45°, triethylamine (453 µl) was added. After 5 min the cooling bath was removed and the reaction mixture was allowed to warm to room temperature during 30 min. The reaction mixture was partitioned between dichloromethane (50 ml) and water (50 ml). The organic phase was separated and the aqueous phase extracted with dichloromethane (25 ml). The combined organic extracts were washed with 2M hydrochloric acid (75 ml), saturated sodium bicarbonate solution (75 ml) and brine (75 ml), and dried ($Na_2SO_4$). The solvent was evaporated and the residue purified by flash chromatography (35 g silica gel, Merck 9385). Elution with ethyl acetate: light petroleum (1:2) afforded the title compound (132mg) as a white foam. $[\alpha]_D^{22}$ + 256° (c0.6, $CHCl_3$); δ ($CDCl_3$) values include 1.76 (3H,s), 1.82 (3H,s), 2.16 (3H,s), 3.40 (2H,m), 5.09 (1H, d, J 9 Hz), 5.52 (2H,m), 6.26 (1H, t, J 8 Hz).

Example 1

(13R)-Hydroxy-23-desoxy Factor A, 5-acetate

Intermediate 3 (4.791g) was added to a stirred mixture of selenium dioxide (416mg) and t-butyl hydroperoxide (3M in dichloromethane; 5ml) in dichloromethane (30ml). After stirring at room temperature for 30h the reaction mixture was diluted with ethyl acetate (200 ml), washed with water and brine, and dried ($Na_2SO_4$). The solvent was evaporated and the residue purified by chromatography (250g silica gel, Merck 9385). Elution with ethyl acetate: light petroleum (1:4→1:2) afforded the title compound (560mg) as a pale yellow foam. $\nu_{max}$($CHBr_3$) 3600, 3460 (OH), 1732 (OAc), 1712 ($CO_2R$), 993$cm^{-1}$ (C-O); δ ($CDCl_3$) values include 0.69 (3H, t, J 5 Hz), 2.15 (3H,s), 3.32 (1H,m), 3.72 (1H, d, J 10 Hz), 4.05 (1H, d, J 5 Hz), 5.52 (2H,m).

Example 2

(13R)-Hydroxy-23-desoxy Factor A

Aqueous sodium hydroxide (1M, 152 μl) was added to a stirred solution of the compound of Example 1 (50mg) in methanol (1 ml) at 0°. After 1.5h at 0° the reaction mixture was diluted with ethyl acetate (50 ml), washed with water and brine and dried ($Na_2SO_4$). The solvent was evaporated and the residue purified by flash chromatography (15 g silica gel, Merck 9385). Elution with ethyl acetate: light petroleum (1:1) afforded the title compound (36mg) as a pale yellow foam. $\nu_{max}$($CHBr_3$) 3560, 3480 (OH), 1708 ($CO_2R$), 990 $cm^{-1}$ (C-O); δ ($CDCl_3$) values include 0.69 (3H, d, J 5 Hz), 3.26 (1H,m), 3.72 (1H, d, J 10 Hz), 3.93 (1H,s), 3.96 (1H, d, J 6 Hz), 4.29 (1H, t, J 6 Hz), 5.30 (3H,m).

Example 3

(13S)-Hydroxy-23-desoxy Factor A, 5-acetate

A solution of sodium borohydride (0.1M in ethanol; 220 μl) was added dropwise to a solution of Intermediate 4 (13 mg) in ethanol (1 ml) at 0°. After 1h at 0° and 30 min at room temperature the reaction mixture was diluted with ethyl acetate (50 ml), washed with 2M hydrochloric acid (50 ml), saturated sodium bicarbonate solution (50 ml) and brine (50 ml), and dried ($Na_2SO_4$). The solvent was evaporated and the residue purified by chromatography (7g silica gel, Merck 9385). Elution with ethyl acetate, light petroleum (1:2) afforded the title compound (10.2mg) as a white foam. δ ($CDCl_3$) values include 0.69 (3H, d, J 5 Hz), 1.53 3H,s), 1.76 (3H,s), 2.16 (3H,s), 3.31 (1H,m), 3.42 (1H, d, J 9 Hz), 5.12 (1H, d, J 9 Hz), 5.35 (2H,m), 5.53 (2H,m), 5.6 to 5.8 (3H,m).

The following are examples of formulations according to the invention. The term 'Active Ingredient' as used hereinafter means a compound of the invention.

Multidose parenteral injection

Example 1

| | % w/v | Range |
|---|---|---|
| Active ingredient | 2.0 | 0.1 - 6.0% w/v |
| Benzyl alcohol | 1.0 | |
| Polysorbate 80 | 10.0 | |
| Glycerol formal | 50.0 | |
| Water for Injections | to 100.0 | |

Dissolve the active ingredient in the polysorbate 80 and glycerol formal. Add the benzyl alcohol and make up to volume with Water for Injections. Sterilize the product by conventional methods, for example sterile filtration or by heating in an autoclave and package aseptically.

Example 2

| | % w/v | Range |
|---|---|---|
| Active ingredient | 4.0 | 0.1 - 7.5% w/v |
| Benzyl alcohol | 2.0 | |
| Glyceryl triacetate | 30.0 | |
| Propylene glycol | to 100.0 | |

Dissolve the active ingredient in the benzyl alcohol and glyceryl triacetate. Add the propylene glycol and make up to volume. Sterilize the product by conventional pharmaceutical methods, for example sterile filtration, and package aseptically.

Example 3

| | % | Range |
|---|---|---|
| Active ingredient | 2.0 w/v | 0.1 - 7.5% w/v |
| Ethanol | 36.0 v/v | |
| Non-ionic surfactant (e.g. Synperonic PE L44*) | 10.0 w/v | |
| Propylene glycol | to 100.0 | |

* Trademark of ICI

Dissolve the active ingredient in the ethanol and surfactant and make up to volume. Sterilize the product by conventional pharmaceutical methods, for example sterile filtration, and package aseptically.

Example 4

| | % | Range |
|---|---|---|
| Active Ingredient | 2.0 w/v | 0.1 - 3.0% w/v |
| Non-ionic surfactant (e.g. Synperonic PE F68*) | 2.0 w/v | |
| Benzyl alcohol | 1.0 w/v | |
| Miglyol 840 ** | 16.0 v/v | |
| Water for Injections | to 100.0 | |

* Trademark of ICI

** Trademark of Dynamit Nobel

Dissolve the active ingredient in the Miglyol 840. Dissolve the non-ionic surfactant and benzyl alcohol in most of the water. Prepare the emulsion by adding the oily solution to the aqueous solution while homogenising using conventional means. Make up to volume. Aseptically prepare and package aseptically.

Aerosol spray

| | % w/w | Range |
|---|---|---|
| Active Ingredient | 0.1 | 0.01 - 2.0% w/w |
| Trichloroethane | 29.9 | |
| Trichlorofluoromethane | 35.0 | |
| Dichlorodifluoromethane | 35.0 | |

Mix the Active Ingredient with trichloroethane and fill into the aerosol container. Purge the headspace with the gaseous propellant and crimp the valve into position. Fill the required weight of liquid propellant under pressure through the valve. Fit with actuators and dust-caps.

Tablet

Method of manufacture - wet granulation

| | mg |
|---|---|
| Active Ingredient | 250.0 |
| Magnesium stearate | 4.5 |
| Maize starch | 22.5 |
| Sodium starch glycolate | 9.0 |
| Sodium lauryl sulphate | 4.5 |
| Microcrystalline cellulose | to tablet core weight of 450mg |

Add sufficient quantity of a 10% starch paste to the active ingredient to produce a suitable wet mass for granulation. Prepare the granules and dry using a tray or fluid-bed drier. Sift through a sieve, add the remaining ingredients and compress into tablets.

If required, film coat the tablet cores using hydroxypropylmethyl cellulose or other similar film-forming material using either an aqueous or non-aqueous solvent system. A plasticizer and suitable colour may be included in the film-coating solution.

Veterinary tablet for small/domestic animal use

Method of manufacture - dry granulation

| | mg |
|---|---|
| Active Ingredient | 50.0 |
| Magnesium stearate | 7.5 |
| Microcrystalline cellulose to tablet core weight of | 75.0 |

Blend the active ingredient with the magnesium stearate and microcrystallise cellulose. Compact the blend into slugs. Break down the slugs by passing through a rotary granulator to produce free-flowing granules.

Compress into tablets.

The tablet cores can then be film-coated, if desired, as described above.

Veterinary intrammary injection

| | | | mg/dose | Range |
|---|---|---|---|---|
| Active Ingredient | | | 150mg | 0.05 - 1.0g |
| Polysorbate 60 | 3.0% w/w) | | ) | |
| White Beeswax | 6.0% w/w) | to 3g | ) | to 3 or 15g |
| Arachis oil | 91.0% w/w) | | ) | |

Heat the arachis oil, white beeswax and polysorbate 60 to 160°C with stirring. Maintain at 160°C for two hours and then cool to room temperature with stirring. Aseptically add the active ingredient to the vehicle and disperse using a high speed mixer. Refine by passing through a colloid mill. Aseptically fill the product into sterile plastic syringes.

Veterinary slow-release bolus

| | % w/w | Range |
|---|---|---|
| Active Ingredient | | 0.25-2g |
| Colloidal silicon | ) | to required |
| dioxide | 2.0) | fill weight |
| Microcrystalline | ) | |
| cellulose | to 100.0) | |

Blend the active ingredient with the colloidal silicon dioxide and microcrystalline cellulose by using a suitable aliquot blending technique to achieve a satisfactory distribution of active ingredient throughout the carrier. Incorporate into the slow release device and give (1) a constant release of active ingredient or (2) a pulsed release of active ingredient.

Veterinary oral drench

| | % w/v | Range |
|---|---|---|
| Active Ingredient | 0.35 | 0.01 - 2% w/v |
| Polysorbate 85 | 5.0 | |
| Benzyl alcohol | 3.0 | |
| Propylene glycol | 30.0 | |
| Phosphate buffer | as pH 6.0 - 6.5 | |
| Water | to 100.0 | |

Dissolve the active ingredient in the Polysorbate 85, benzyl alcohol and the propylene glycol. Add a proportion of the water and adjust the pH to 6.0 - 6.5 with phosphate buffer, if necesssary. Make up to final volume with the water. Fill the product into the drench container.

Veterinary oral paste

| | % w/w | Range |
|---|---|---|
| Active Ingredient | 4.0 | 1 - 20% w/w |
| Saccharin sodium | 2.5 | |
| Polysorbate 85 | 3.0 | |
| Aluminium distearate | 5.0 | |
| Fractionated coconut oil | to 100.0 | |

Disperse the aluminium distearate in the fractionated coconut oil and polysorbate 85 by heating. Cool to room temperature and disperse the saccharin sodium in the oily vehicle. Disperse the active ingredient in the base. Fill into plastic syringes.

Granules for veterinary in-feed administration

| | % w/w | Range |
|---|---|---|
| Active Ingredient | 2.5 | 0.05-5% w/w |
| Calcium sulphate, hemi-hydrate | to 100.0 | |

Blend the Active Ingredient with the calcium sulphate. Prepare the granules using a wet granulation process. Dry using a tray or fluid-bed drier. Fill into the appropriate container.

Veterinary Pour-on

| | % w/v | Range |
|---|---|---|
| Active Ingredient | 2.0 | 0.1 to 30% |
| Dimethyl sulphoxide | 10.0 | |
| Methyl Isobutyl ketone | 30.0 | |
| Propylene glycol (and pigment) | to 100.0 | |

Dissolve the active ingredient in the dimethyl sulphoxide and the methyl isobutyl ketone. Add the pigment and make up to volume with the propylene glycol. Fill into the pour-on container.

Emulsifiable Concentrate
Active ingredient 50g
Anionic emulsifier (e.g. Phenyl sulphonate CALX) 40g
Non-ionic emulsifier (e.g. Synperonic NP 13) (Trademark of ICI) 60g
Aromatic solvent (e.g. Solvesso 100) to 1 litre.
Mix all ingredients, stir until dissolved.

Granules

(a) Active ingredient 50g
Wood resin 40g

Gypsum granules (20-60 mesh) to 1kg (e.g. Agsorb 100A)
(b) Active ingredient 50g
Synperonic NP13 (Trademark of ICI) 40g
Gypsum granules (20-60 mesh) to 1kg.

Dissolve all ingredients in a volatile solvent e.g. methylene chloride, add to granules tumbling in mixer. Dry to remove solvent.

## Claims

1. Compounds of formula (I)

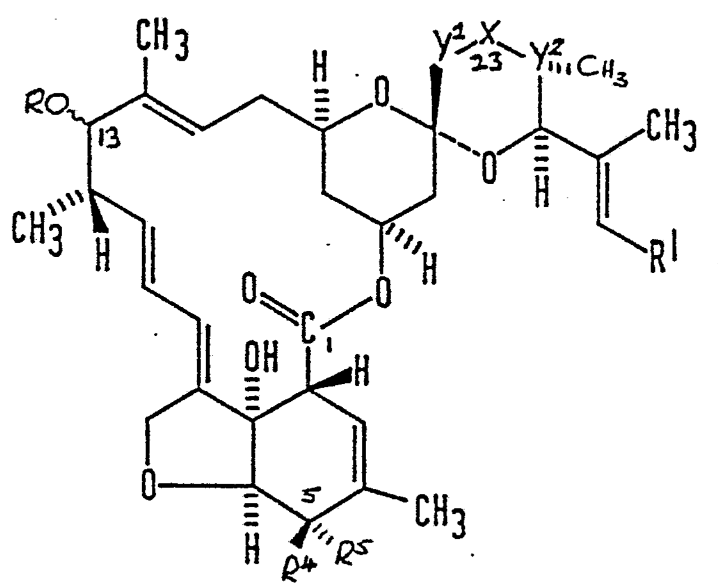

(I)

wherein R represents a hydrogen atom or a silyl group;
$R^1$ represents a methyl, ethyl or isopropyl group;
$Y^1$ is $-CH_2-$, $Y^2$ is -CH- and X represents $-C(R^2)(R^3)$-[where $R^2$ represents a hydrogen atom or a group $OR^6$ (where $OR^6$ is a hydroxy group or a substituted hydroxyl group having up to 25 carbon atoms) and $R^3$ represents a hydrogen atom, or $R^2$ and $R^3$ together with the carbon atom to which they are attached represents C=O, $C=CH_2$ or $C=NOR^7$ (where $R^7$ represents a hydrogen atom or a $C_{1-8}$ alkyl group and the group $C=NOR^7$ is the E configuration)] or $-Y^1-X-Y^2-$ represents -CH=CH-CH- or $-CH_2-C=C-$;
$R^4$ represents a group $OR^6$ as defined above and $R^5$ represents a hydrogen atom or $R^4$ and $R^5$ together with the carbon atom to which they are attached represents C=O.

2. Compounds according to claim 1 in which $R^1$ is an isopropyl group.

3. Compounds according to claim 1 or claim 2 in which $R^4$ is an hydroxy or acetoxy group.

4. Compounds according to claim 1 in which R is a hydrogen atom, $R^1$ is an isopropyl group, $Y^1$ is $-CH_2-$, $Y^2$ is -CH-, X is $-C(R^2)(R^3)$, in which $R^2$ is a hydrogen atom or a hydroxy, ethoxy or acetyloxy group and $R^3$ is hydrogen atom, or $R^2$ and $R^3$ together with the carbon atom to which they are attached represents C=O, $C=CH_2$ or $C=NOCH_3$ (where the group $C=NOCH_3$ is in the E configuration); and $R^4$ is a hydroxy, methoxy or acetoxy group and $R^5$ is a hydrogen atom.

5. The compound according to claim 1 in which R is a hydrogen atom, $R^1$ is an isopropyl group, $Y^1$ is $-CH_2-$, $Y^2$ is -CH-, X is $-CH_2-$, $R^4$ is a hydroxyl or acetoxy group and $R^5$ is a hydrogen atom.

6. A composition for use in human medicine containing an effective amount of at least one compound according to claim 1 together with one or more carriers and/or excipients.

7. A composition for use in veterinary medicine containing an effective amount of at least one compound according to claim 1 together with one or more carriers and/or excipients.

8. A pest control composition containing an effective amount of at least one compound according to claim 1 together with one or more carriers and/or excipients.

9. A method for combatting pests in agriculture, horticulture or forestry, or in stores, buildings or other public places or locations of the pests, which comprises applying to plants or other vegetation or to the pests themselves or a location thereof an effective amount of one or more compounds according to claim

1.

10. A process for the preparation of a compound according to claim 1 which comprises:

(A) in the preparation of a compound of formula (I) in which the 13-hydroxyl group is in the (R)-configuration and $R^4$ is as defined for formula (I) but is not a hydroxyl or silyloxy group, oxidising the corresponding 13-desoxy compound;

(B). in the preparation of a compound of formula (I) in which the 13-hydroxyl group is in the (S)-configuration, reducing the corresponding 13-oxo compound;

(C) in the preparation of a compound of formula (I) in which $R^4$ is a hydroxyl group, converting a corresponding compound of formula (I) in which $R^4$ is a substituted hydroxyl group into the desired unsubstituted hydroxy compound;

(D) in the preparation of a compound of formula (I) in which $R^4$ is a substituted hydroxyl group, reacting a corresponding compound of formula (I) in which $R^4$ is a hydroxyl group with a reagent for converting a hydroxyl group into a substituted hydroxyl group;

(E) in the preparation of a compound of formula (I) in which X is $-C(R^2)R^3)-$ and $R^2$ and $R^3$ together with the carbon atom to which they are attached represents $C=O$ and/or $R^4$ and $R^5$ together with the carbon atom to which they are attached represents $C=O$, oxidising a corresponding compound wherein $R^2$ is a hydroxyl group and $R^3$ is a hydrogen atom and/or $R^4$ is a silyloxy group and $R^5$ is a hydrogen atom;

(F) in the preparation of a compound of formula (I) in which X is $-C(R^2)(R^3)-$ and $R^2$ and $R^3$ together with the carbon atom to which they are attached represent $C=NOR^7$, reacting the corresponding 23-keto compound with a reagent $H_2NOR^7$ (where $R^7$ is as defined in claim 1); or

(G) in the preparation of a compound of formula (I) in which X is a group $C=CH_2$, reacting a corresponding compound of formula (I) in which X is $C=O$ with a phosphorane of formula $(R^{11})_3P=CH_2$ (where $R^{11}$ is $C_{1-6}$ alkyl or phenyl).

11. The 13-oxo analogues of the compounds of claim 1.